# EUROPEAN PATENT SPECIFICATION

(11) **EP 4 378 493 B1**
(45) Date of publication and mention of the grant of the patent: **19.08.2026**
(21) Application number: 22864277.3
(22) Date of filing: 18.08.2022
(51) Int. Cl.: A61B 17/072, A61L 31/14, A61B 17/115, A61L 31/04, A61B 17/00, A61B 90/00

(54) **MEDICAL MEMBER**
MEDIZINISCHES ELEMENT
ÉLÉMENT MÉDICAL

(30) Priority: 03.09.2021 JP 2021143979
(43) Date of publication of application: 05.06.2024
(73) Proprietor: TERUMO Kabushiki Kaisha, Tokyo 151-0072 (JP)
(72) Inventor: ARAMAKI, Naoki, Ashigarakami-gun, Kanagawa 259-0151 (JP); KAI, Miho, Ashigarakami-gun, Kanagawa 259-0151 (JP); SHIRAISHI, Mizuho, Ashigarakami-gun, Kanagawa 259-0151 (JP); SUZUKI, Miria, Ashigarakami-gun, Kanagawa 259-0151 (JP)
(74) Representative: Casalonga
(86) International application number: PCT/JP2022/031176
(87) International publication number: WO 2023/032692

(56) References cited:
- WO-A1-2020/067380
- WO-A1-2020/196856
- WO-A1-2020/196857
- JP-A- 2004 147 969
- US-A1- 2020 360 002
- US-A1- 2021 204 949

## Description

### Technical Field

The present invention relates to a medical member applied to an anastomotic portion of a biological organ. In particular, the present invention relates to a medical member according to the preamble of independent claim 1, such as it is e.g. known from US 2021/0204949 A1.

### Background Art

In the medical field, a technique of joining biological organs by surgery (for example, an anastomosis for joining alimentary canals) is known. It is also known that, when the above-described technique is performed, it is important as a postoperative prognostic determinant that delay in adhesion does not occur at a joint (hereinafter, also referred to as "anastomotic portion") where biological organs are joined to each other.

Various methods and medical tools are used in the technique of joining biological organs, and for example, a method for suturing a biological organ with a biodegradable suture thread and a method using a mechanical anastomosis device (see Patent Literature 1) for performing anastomosis with a stapler have been proposed. In particular, when an anastomosis is performed using a mechanical anastomosis device, it is possible to enhance a joining force between biological organs at an anastomotic portion as compared with a method using a suture thread, and thus, it is possible to reduce a risk of suture failure.

However, the degree of progress of adhesion at an anastomotic portion also depends on a state of a biological tissue at an anastomosis target site of a patient, and the like. Therefore, for example, even when an anastomosis device as described in Patent Literature 1 is used, there is a possibility that a risk of suture failure cannot be sufficiently reduced depending on a state of a biological tissue of a patient.

In order to cope with the problems as described above, use of a medical member described in Patent Literature 2 below has been proposed in an anastomosis for joining biological organs.

The medical member described in Patent Literature 2 is made of a sheet-like member having a through-hole. When the medical member is indwelled in a state of being sandwiched between biological organs to be anastomosed, the medical member promotes adhesion of an anastomotic portion by accumulating a biological component in the through-hole. Therefore, it is possible to effectively enhance a joining force at an anastomotic portion by performing an anastomosis using the medical member.

### Citation List

### Patent Literature

Patent Literature 1: JP 2007-505708 A
Patent Literature 2: WO 2019/156230 A

### Summary of Invention

### Technical Problem

In the technique using the medical member, the shape and size of the medical member may be processed at a medical site according to a biological organ to be anastomosed. For example, when an alimentary canal including a lumen is selected as a biological organ to be joined, a surgeon presses a cutter of a stapler against a medical member in a direction (thickness direction) intersecting with a plane direction in a state where the medical member is disposed between one alimentary canal and the other alimentary canal, and punches out the medical member to form an opening communicating with the lumen of the alimentary canal.

When the surgeon performs breaking operation (punching) as described above, if the medical member cannot be smoothly broken with the cutter of the stapler, the technique is complicated. In addition, "punching failure" in which fraying occurs in the medical member may occur.

As described above, the medical member may require breakability (hereinafter, referred to as "breaking processability") in a direction intersecting with a plane direction depending on its use. In addition to the breaking processability, the medical member also requires flexibility for being able to follow movement of a biological organ in a state where the medical member is indwelled in a living body, and a predetermined strength for facilitating handling when a surgeon (a medical worker including the surgeon) operates the medical member.

The inventors of the present invention have found that it is possible to improve the breaking processability of the medical member by reducing the flexibility and strength of the medical member on the basis of the following findings.

When the flexibility of the medical member is low, it is difficult to extend the medical member. Therefore, when a cutter is pressed against the medical member, a force of pressing the cutter can be sufficiently transmitted to the medical member. As a result, the medical member can be easily broken. In addition, in a case where the strength of the medical member is low, when a cutter is pressed against the medical member, the medical member can be easily bitten by the cutter. Therefore, the medical member can be easily broken.

However, when the "flexibility" and the "strength" are reduced in order to improve the "breaking processability" of the medical member, the medical member is deteriorated in followability to a biological organ and handleability at the time of handling. Therefore, applicability of the medical member to an anastomosis is reduced.

The inventors have found that presence or absence of occurrence of punching failure largely depends on the physical properties of the medical member. Then, in order to solve such a problem, the inventors have invented a medical member having effectively improved breaking processability while having appropriate flexibility and strength required at the time of application to an anastomosis.

An object of the present invention is to provide a medical member having flexibility and strength suitable for application to an anastomotic portion, and having effectively improved breaking processability.

### Solution to Problem

The above problem is solved by a medical member according to independent claim 1. The dependent claims relate to advantageous embodiments.

### Advantageous Effects of Invention

According to an embodiment of the present invention, it is possible to provide a medical member having appropriate flexibility and strength for application to an anastomotic portion and having effectively improved breaking processability.

### Brief Description of Drawings

Fig. 1 is a perspective view of a medical member according to an embodiment of the present invention.
Fig. 2 is a partial cross-sectional view of the medical member taken along line 2-2 illustrated in Fig. 1.
Fig. 3 is a plan view of the medical member.
Fig. 4 is a diagram for explaining a test method of a punching test 1 of Example.
Fig. 5 is a diagram for explaining a test method of a punching test 2 of Example.
Fig. 6 is a diagram schematically illustrating a part of a sample medical member (sample 100A) used in Example.
Fig. 7 is a diagram schematically illustrating a part of a sample medical member (sample 100B) used in Example.
Fig. 8 is a diagram schematically illustrating a part of a sample medical member (sample 100C) used in Example.
Fig. 9 is a diagram schematically illustrating a part of a sample medical member (sample 100D) used in Example.
Fig. 10 is a diagram schematically illustrating a part of a sample medical member (sample 100E) used in Example.
Fig. 11 is a chart illustrating a relationship between a compression elongation percentage and a breaking strength of a medical member obtained in Example.
Fig. 12 is a flowchart illustrating steps of a treatment method using a medical member.
Fig. 13 is a flowchart illustrating steps of an embodiment of a treatment method (large intestine anastomosis).
Fig. 14 is a schematic cross-sectional view for explaining a large intestine anastomosis.
Fig. 15 is a schematic cross-sectional view for explaining a large intestine anastomosis.
Fig. 16 is a schematic cross-sectional view for explaining a large intestine anastomosis.

### Description of Embodiment

### (Embodiment)

Hereinafter, an embodiment of the present invention will be described with reference to the attached drawings. Note that, in the description of the drawings, the same elements are denoted by the same reference numerals, and redundant description will be omitted. In addition, dimensional ratios of the drawings may be exaggerated for convenience of description, and may be different from actual ratios.

### <Medical member>

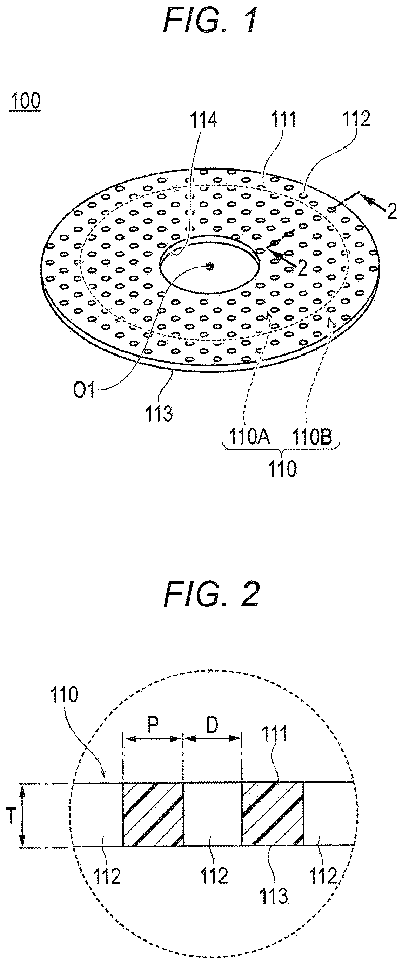

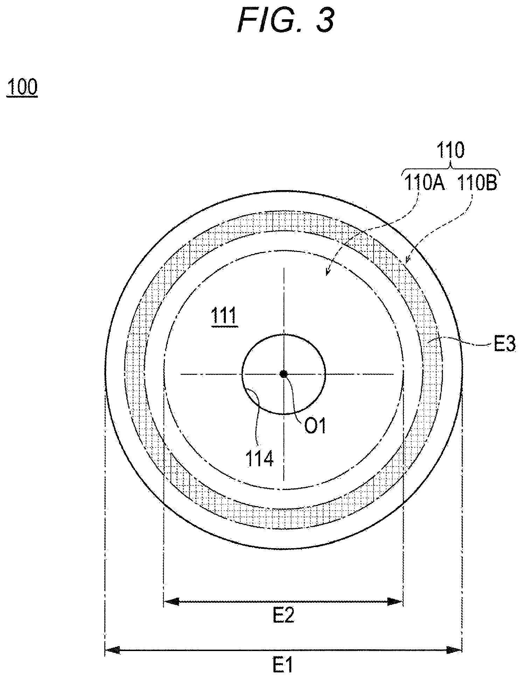

Fig. 1 is a perspective view illustrating a medical member 100 according to the present embodiment. Fig. 2 is an enlarged cross-sectional view illustrating a part of the medical member 100 taken along line 2-2 illustrated in Fig. 1. Fig. 3 is a plan view of the medical member 100 as viewed from a front surface 111 side.

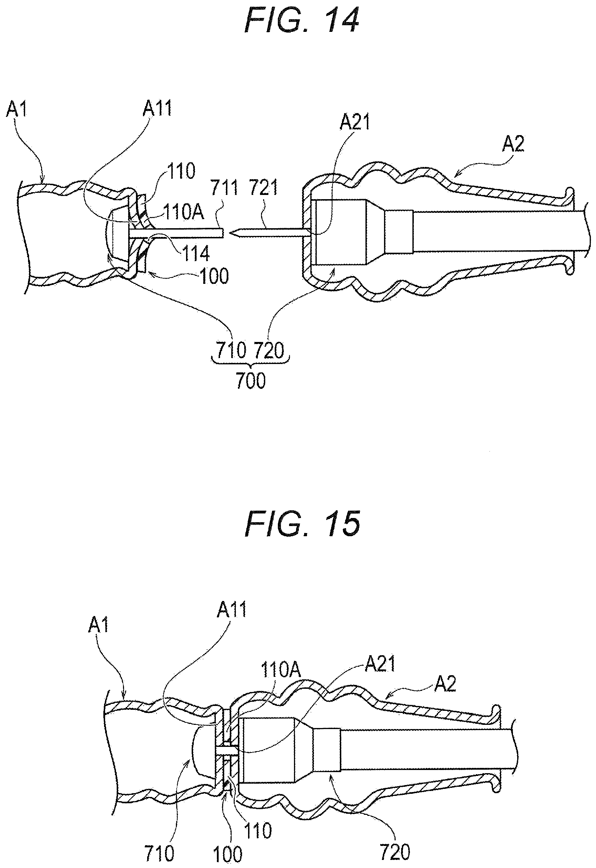

As illustrated in Figs. 14 to 16, the medical member 100 can be applied to a technique of joining predetermined biological organs (for example, an anastomosis of an alimentary canal). As described later, in the description of the present specification, a large intestine anastomosis will be described as a technique example using the medical member 100.

The medical member 100 has a function as an adhesion-promoting device that promotes adhesion of biological tissues of two biological organs by being indwelled between two or more biological organs to be anastomosed.

Specifically, the medical member 100 induces expression of a biological component of a biological organ by being applied to an anastomotic portion of the biological organ. The medical member 100 can promote adhesion by the induced biological component passing through through-holes 112 and being accumulated.

### <Main body>

As illustrated in Figs. 1 and 3, the medical member 100 includes a mesh-like main body 110 having the plurality of through-holes 112.

The plurality of through-holes 112 passes from the front surface 111 of the main body 110 to a back surface 113 of the main body 110 in a thickness direction (up-down direction in Fig. 2) of the main body 110. Note that, in the present specification, the same reference numeral 112 is used for "a plurality of through-holes" and "through-hole" for convenience of description.

The main body 110 can be made of a sheet-like member having a circular shape in plan view illustrated in Fig. 3. Note that the planar shape of the main body 110 is not particularly limited, and may be, for example, an ellipse or a polygon (a rectangle, a triangle, or the like).

The main body 110 has a hole portion 114 formed in a predetermined range including a center portion O1 of the main body 110 in a plane direction.

An engaged portion 711 of a first engagement tool 710 of a joining device 700 described later can be inserted into the hole portion 114 (see Fig. 14). A surgeon can cause the first engagement tool 710 to hold the medical member 100 by inserting the engaged portion 711 into the hole portion 114.

As illustrated in Fig. 1, the through-holes 112 formed in the main body 110 are regularly and periodically formed in the plane direction of the main body 110. Note that the through-holes 112 may be randomly formed in portions in the plane direction of the main body 110.

As illustrated in Fig. 2, each of the through-holes 112 extends substantially perpendicularly between the front surface 111 and the back surface 113 in a thickness direction (up-down direction in Fig. 2) of the main body 110. Note that each of the through-holes 112 may be bent in a zigzag shape or curved between the front surface 111 and the back surface 113 in a cross section in the thickness direction of the main body 110.

Each of the through-holes 112 has a substantially circular planar shape. Note that the planar shape of each of the through-holes 112 is not particularly limited, and may be, for example, an ellipse or a polygon (a rectangle, a triangle, or the like), an irregular planar shape, or the like. The planar shape or cross-sectional shape may be different among the through-holes 112.

The thickness (dimension T illustrated in Fig. 2) of the main body 110 is not particularly limited, but is preferably 0.05 to 0.3 mm, and more preferably 0.1 to 0.2 mm. When the thickness of the main body 110 is 0.05 mm or more (particularly, 0.1 mm or more), the main body 110 can have strength to such an extent that the main body 110 is not damaged when the medical member 100 is handled. Meanwhile, when the thickness of the main body 110 is 0.3 mm or less (particularly, 0.2 mm or less), the main body 110 can have sufficient flexibility for the main body 110 to closely adhere to a biological tissue to which the main body 110 is applied and to follow the biological tissue.

In the main body 110, for example, a value of a ratio of a hole diameter D (distance D illustrated in Fig. 2) of the through-hole 112 to a pitch P of the through-holes 112 (distance P illustrated in Fig. 2, distance between adjacent through-holes 112, corresponding to a "wire diameter" in Example described later) is preferably 0.25 or more and less than 40. Note that when the planar shape of the through-hole 112 is a perfect circle, the hole diameter D of the through-hole 112 is equal to a diameter of the perfect circle. Meanwhile, when the planar shape of the through-hole 112 is not a perfect circle, a diameter (diameter equivalent to a circle) of a perfect circle having the same area as the area of an opening of the through-hole 112 (portion facing the front surface 111 or the back surface 113 in the through-hole 112) can be defined as the hole diameter D of the through-hole 112.

Since the main body 110 has the plurality of through-holes 112, there is a plurality of values of the hole diameter D corresponding to the through-holes 112. Therefore, in the present embodiment (the same applies to samples 100A to 100E described in Example described later), when a value of the above-described ratio is calculated, an arithmetic average value of two or more values of the hole diameter D corresponding to the plurality of through-holes 112 is used as a representative value of the hole diameter D. Meanwhile, the pitch P of the plurality of through-holes 112 is defined by the shortest distance between openings of two through-holes 112. Note that, also as for a value of the pitch P, there is a plurality of values of the pitch P corresponding to combinations of adjacent through-holes 112. Therefore, in the present embodiment, when a value of the above-described ratio is calculated, an arithmetic average value of two or more values of the pitch P corresponding to combinations of adjacent through-holes 112 is used as a representative value of the pitch P.

Note that the pitch P of the through-holes 112, the hole diameter D of the through-hole 112, the ratio of the hole diameter D to the pitch P, and the like are merely examples, and the present invention is not limited thereto.

The main body 110 can be made of, for example, a biodegradable material. The constituent material of the main body 110 is not particularly limited, and examples thereof include a biodegradable resin. As the biodegradable resin, for example, known biodegradable (co)polymers such as those described in JP 2011-528275 A, JP 2008-514719 A, WO 2008 -1952 A, and JP 2004-509205 A can be used. Specific examples thereof include: (1) a polymer selected from the group consisting of an aliphatic polyester, a polyester, a polyacid anhydride, a polyorthoester, a polycarbonate, a polyphosphazene, a polyphosphoric acid ester, a polyvinyl alcohol, a polypeptide, a polysaccharide, a protein, and a cellulose; and (2) a copolymer containing one or more monomers constituting the (1). That is, the biodegradable sheet preferably contains at least one biodegradable resin selected from the group consisting of a polymer selected from the group consisting of an aliphatic polyester, a polyester, a polyacid anhydride, a polyorthoester, a polycarbonate, a polyphosphazene, a polyphosphoric acid ester, a polyvinyl alcohol, a polypeptide, a polysaccharide, a protein, and a cellulose, and a copolymer containing one or more monomers constituting the polymer.

A method for manufacturing the main body 110 is not particularly limited, and examples thereof include a method for manufacturing fibers made of the above-described biodegradable resin and manufacturing a mesh-like sheet using the fibers. The method for manufacturing fibers made of the biodegradable resin is not particularly limited, and examples thereof include an electrospinning method (electrospinning method/electrostatic spinning method) and a melt blowing method. For the main body 110, only one of the above methods may be selected and used, or two or more thereof may be selected and appropriately combined. Note that still other examples of the method for manufacturing the main body 110 include a method for manufacturing the biodegradable sheet according to the present invention by spinning fibers made of the above-described biodegradable resin according to a usual method and knitting the obtained fibers into a mesh shape, a method for manufacturing the biodegradable sheet by compressing the fibers, and a method for manufacturing the biodegradable sheet by entangling the fibers without weaving the fibers.

The main body 110 causes a biological reaction with a constituent material such as a biodegradable resin. The main body 110 induces expression of a biological component such as fibrin by this action. The biological component induced in this manner can be accumulated so as to pass through the through-holes 112 of the main body 110, thereby promoting adhesion. Therefore, by disposing the main body 110 of the medical member 100 between biological organs to be joined, adhesion by the above mechanism is promoted.

Note that the material quality of the main body 110 does not have to be biodegradable as long as adhesion can be promoted.

The hole portion 114 formed in the main body 110 has a larger hole diameter than each of the through-holes 112. The hole portion 114 is formed in a range including the center portion O1 (a center position on a plan view illustrated in Fig. 3) of the main body 110. Note that the center portion O1 is a rotation center of the main body 110 when the main body 110 has a rotationally symmetric shape.

The hole portion 114 has a circular planar shape. The hole diameter of the hole portion 114 can be formed to be, for example, 5 mm to 25 **mm.** Note that the planar shape of the hole portion 114 is not particularly limited, and may be, for example, an ellipse or a polygon (a rectangle, a triangle, or the like). The size of the hole portion 114 is not particularly limited, either.

The hole portion 114 may be formed in advance in the main body 110, or may be formed by a surgeon while an anastomosis is performed. In addition, the surgeon can select various modifications regarding the shape, structure, and the like of the main body 110 according to progress of the technique and the like.

The main body 110 includes a breakable portion 110A and a pressure resistance improving portion 110B.

As illustrated in Figs. 1 and 3, the breakable portion 110A can be formed in a part or the whole of the main body 110.

At least a part of the breakable portion 110A can be disposed at a position closer to the center portion O1 of the main body 110 in the plane direction than an outer peripheral portion of the main body 110.

The "outer peripheral portion of the main body 110" means an arbitrary range located along an outer peripheral edge of the main body 110 and directed from the outer peripheral edge toward the center portion O1.

The breakable portion 110A is disposed so as to surround a periphery of the hole portion 114 formed in a range including the center portion O1 of the main body 110.

The breakable portion 110A has a physical property of easily inducing breakage of the main body 110 when a force is applied in a direction intersecting with the plane direction of the main body 110 (for example, a direction perpendicular to the front surface 111 and the back surface 113 of the main body 110). Therefore, when punching out the main body 110 in the direction intersecting with the plane direction with the joining device 700, a surgeon can easily punch out the main body 110 by causing the breakable portion 110A to be included in a punching target site (see Fig. 15). Note that details of physical properties and the like of the breakable portion 110A will be described in Example described later.

The pressure resistance improving portion 110B is disposed closer to an outer peripheral portion of the main body 110 than the breakable portion 110A so as to surround a periphery of the breakable portion 110A.

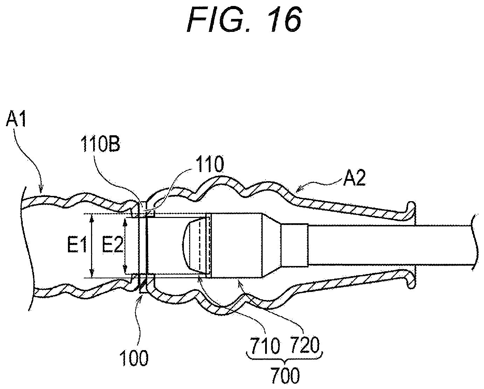

The pressure resistance improving portion 110B has a function of improving sealability between biological organs to be joined when being sandwiched between the biological organs and indwelled (see Fig. 16).

The function of the pressure resistance improving portion 110B depends on a breaking strength of the pressure resistance improving portion 110B in the plane direction. Therefore, when the pressure resistance improving portion 110B is disposed in the main body 110, a predetermined portion (region) of the main body 110 can be processed in order to enhance a breaking strength, or a material having a high breaking strength can be selected as a material constituting the predetermined portion (region).

With reference to the plan view of Fig. 3, a positional relationship between the portions 110A and 110B of the main body 110 and portions of the joining device 700 used for an anastomosis will be described. Note that, in Fig. 3, the through-holes 112 are not illustrated.

A first region E1 is a region where a first engagement tool 710 and a second engagement tool 720 of the joining device 700 overlap each other.

A second region E2 is a portion to be punched out by the joining device 700 in order to form an opening communicating with a lumen of a biological organ (for example, large intestine) to be anastomosed.

At least a part of the breakable portion 110A formed in the main body 110 can be disposed at a position overlapping the second region E2. Each of the engagement tools 710 and 720 included in the joining device 700 includes a cutter (blade). When joining biological organs, the joining device 700 engages the engagement tools 710 and 720 with each other, and punches out the breakable portion 110A at the same time as suturing (see Figs. 15 and 16).

A third region E3 (shaded portion in Fig. 3) corresponds to a joint portion that is indwelled in a state of being sandwiched between biological organs when the biological organs are joined to each other by the engagement tools 710 and 720. The third region E3 promotes adhesion of an anastomotic portion by being indwelled between the biological organs. In the technique using the joining device 700, a joining member (for example, staples) is supplied to the third region E3, and the third region E3 is joined to the biological organs. Therefore, in the medical member 100, the pressure resistance improving portion 110B capable of improving sealability of the anastomotic portion is preferably disposed in at least a part of the third region E3.

In the medical member 100, in consideration of the structure of the joining device 700 used in an anastomosis, the breakable portion 110A having enhanced breaking processability is disposed in a predetermined range surrounding the periphery of the center portion O1 of the main body 110, and the pressure resistance improving portion 110B that improves the sealability of the anastomotic portion is disposed in a predetermined range on an outer peripheral side surrounding a periphery of the breakable portion 110A.

Note that a specific structure of the medical member 100 is not limited as long as the breakable portion 110A is formed in at least a part of the mesh-like main body 110.

For example, the pressure resistance improving portion 110B does not have to be disposed in the main body 110. A position where the breakable portion 110A is disposed in the main body 110 is not limited only to a region including the center portion O1 of the main body 110. For example, the breakable portion 110A can be formed in a region including an outer peripheral portion of the main body 110 or in an arbitrary region between the center portion O1 and the outer peripheral portion.

For example, a reinforcing portion that prevents deformation such as twisting of the main body 110 may be disposed in the outer peripheral portion of the main body 110. The reinforcing portion can be made of, for example, a material harder than the breakable portion 110A and the pressure resistance improving portion 110B.

### <Breakable portion>

The breakable portion 110A according to the present embodiment has the following physical properties. Specifically,
when a breaking strength of the main body 110 in a direction intersecting with a plane direction is represented by Y [N], and
a compression elongation percentage of the main body 110 in the plane direction is represented by X [%],
a relationship between the breaking strength and the compression elongation percentage is represented by the following formulas (1) to (4): $Y \leq 244734 {\left(X-Z\right)}^{- 1.665}$ $0 \leq Z < 408.216$ $X - Z > 0$ $39 \leq X$

The breakable portion 110A is configured such that the breaking strength and the compression elongation percentage satisfy the above formulas (1) to (4), and therefore breaking processability required at the time of forming an anastomotic portion is favorable. In addition, the breakable portion 110A having such a physical property can have appropriate strength capable of ensuring handleability when a surgeon operates the main body 110 as well as the breaking processability suitable for an anastomosis. Furthermore, the breakable portion 110A has appropriate flexibility capable of exhibiting high followability to movement of a joined biological organ in a state of being indwelled in the anastomotic portion.

In the formula (2), the breakable portion 110A preferably satisfies 0 ≤ Z ≤ 351.466, and more preferably satisfies Z = 0. In the breakable portion 110A, when Z satisfies the above condition in the above formula (2), breaking processability is more effectively enhanced.

Note that the relationship between the breaking strength and the compression elongation percentage represented by the above formulas (1) to (4), definitions of physical properties, specific effects of the breakable portion 110A described below, and the like will be described in detail through Example described later.

The breaking strength of the breakable portion 110A can be, for example, 16 N or less. When the breaking strength of the breakable portion 110A is 16 N or less, breaking processability of the breakable portion 110A is effectively enhanced. Note that the breakable portion 110A more preferably has a breaking strength of 0.2 N or more and 16 N or less from a viewpoint of improving the breaking processability and preventing handleability of a surgeon from being significantly impaired in a technique using the medical member 100.

The compression elongation percentage of the breakable portion 110A can be, for example, 1414% or less. When the compression elongation percentage of the breakable portion 110A is 1414% or less, extension of the breakable portion 110A in the plane direction can be effectively suppressed when the breakable portion 110A is punched out. Therefore, the breaking processability of the breakable portion 110A is more effectively improved. Note that the breakable portion 110A more preferably has a compression elongation percentage of 39% or more and 1312% or less from a viewpoint of further improving the breaking processability.

A constituent material of the breakable portion 110A preferably contains polyglycolic acid. The breakable portion 110A exhibits favorable breaking processability by containing polyglycolic acid as a constituent material.

The breakable portion 110A can be made of a nonwoven fabric containing polyglycolic acid as a constituent material. The breakable portion 110A configured in this manner has favorable breaking processability and biodegradability. Therefore, applicability to an anastomosis in which a biological organ is anastomosed is improved.

The breakable portion 110A can be constituted by a heated portion obtained by subjecting a nonwoven fabric containing polyglycolic acid as a constituent material to heat treatment. Since the breakable portion 110A is constituted by a heated portion, the medical member 100 has favorable breaking processability.

### (Example)

Hereinafter, Example of the present invention will be described. Note that the claims of the present invention are not limited to the contents of Example described below.

### <Punching test>

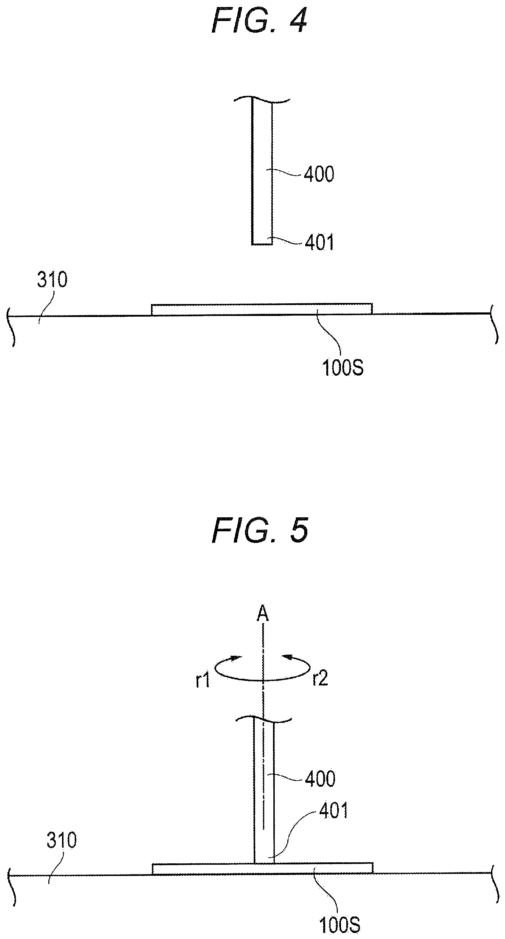

A punching test was performed on a plurality of sample medical members 100S. The punching test was performed by a method illustrated in Figs. 4 and 5.

### <Punching test 1>

In a punching test 1 illustrated in Fig. 4, the sample medical member 100S was placed on a base 310 made of polyethylene, and a distal end (cutting edge having φ of 6 mm) 401 of a biopsy trepan 400 known in the medical field was brought close to and pressed against the sample medical member 100S in a state of being parallel to a base surface of the base 310. That is, the distal end 401 of the biopsy trepan 400 was pressed against the sample medical member 100S in a flat posture from a direction perpendicular to the plane direction of the sample medical member 100S. The number of times the biopsy trepan 400 was pressed against the sample medical member 100S was only one.

### <Punching test 2>

In a punching test 2 illustrated in Fig. 5, the sample medical member 100S was placed on the base 310 made of polyethylene, and the distal end (cutting edge having φ of 6 mm) 401 of the biopsy trepan 400 was brought close to and pressed against the sample medical member 100S in a state of being parallel to the base surface of the base. Thereafter, the biopsy trepan 400 was twisted once in each of an r1 direction and an r2 direction in Fig. 5 around a reference axis A parallel to an axial direction of the biopsy trepan 400 while maintaining the pressed state.

In the present Example, only the punching test 1 or the punching test 1 and the punching test 2 were performed on the plurality of types of sample medical members 100S. Hereinafter, test results of the punching tests 1 and 2 performed on each of the sample medical members 100S will be described with reference to Tables 1 to 5.

### <Samples A1 to A15>

Table 1 presents punching test results of medical members as samples A1 to A15.

The following medical members were prepared as samples A1 to A15. In the following description, samples A1 to A15 are collectively referred to as "sample 100A".

### <Sample 100A>

- A sheet including the mesh-like main body 110 made of a polyglycolic acid (PGA) nonwoven fabric was prepared as the sample 100A.
- The sheet was constituted by two sheets made of a coarse nonwoven fabric. The two sheets were disposed to overlap each other in a thickness direction. Each of the sheets includes through-holes of random shapes, formed between fibers constituting the sheet. The through-holes of the two sheets pass from a front surface to a back surface at a position where the through-holes overlap each other in the plane direction. This passing portion constitutes the through-hole 112 of the medical member 100.

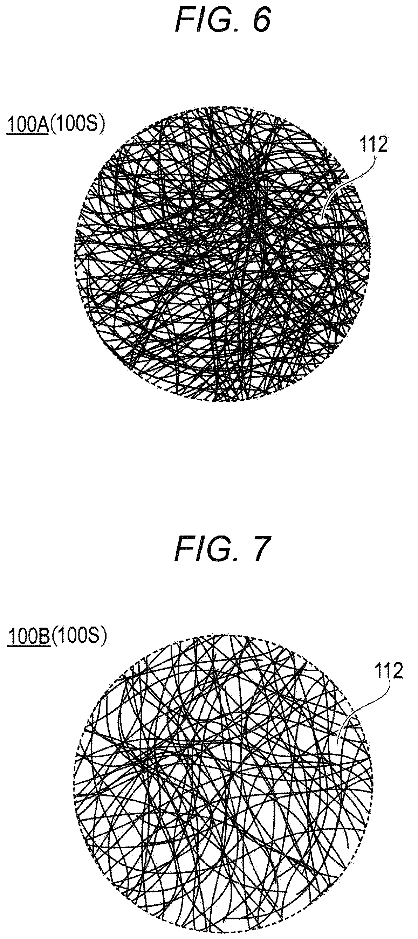

- Fig. 6 illustrates a schematic view in which a part of the sample 100A is enlarged. In the sample 100A, the through-hole 112 has a hole diameter of 0.2 mm to 0.8 mm and a wire diameter (pitch) of 0.1 mm to 0.2 mm. The sample 100A has a thickness of 0.1 mm to 0.2 mm.
- A heated portion that had been subjected to heat treatment in the main body 110 of the sample 100A or an arbitrary portion that had not been subjected to heat treatment in the main body 110 of the sample 100A was defined as the breakable portion 110A. Each of the punching tests was performed on the breakable portion 110A. Conditions of the heat treatment for forming the breakable portion 110A are as follows.
   (1) A hot pressing machine was pressed against the front surface 111 of the main body 110 to perform hot pressing.
   (2) A pressing pressure was set to 20 MPa or 40 MPa.
   (3) A pressing time was set to 30 minutes.
   (4) A heating temperature was set to 90°C to 220°C.

### <Evaluation method>

- A sample that could not be punched out in both the punching test 1 and the punching test 2 was evaluated as × in comprehensive evaluation. It can be determined that the sample evaluated as × in the comprehensive evaluation is not suitable for the anastomosis assumed in the present invention.
- When a sample could be punched out in the punching test 1, the sample was evaluated as ∘ in evaluation of the punching test 1. Note that the above determination that "a sample could be punched out" was made on the basis of whether or not the distal end 401 of the biopsy trepan 400 could pass through the main body 110 of the sample medical member 100S in the thickness direction.
- When a sample could be punched out in the punching test 2, the sample was evaluated as ∘ in evaluation of the punching test 2.
- In the comprehensive evaluation, a sample that could be punched out in the punching test 2 was evaluated as o. In addition, a sample that could be punched out in the punching test 1 was evaluated as ⊙. In the punching test 2, since the biopsy trepan 400 is twisted in a state where the biopsy trepan 400 is pressed against the sample medical member 100S (see Fig. 5), the sample medical member 100S can be punched out more easily than in the punching test 1 in which the biopsy trepan 400 is simply pressed against the sample medical member 100S. Therefore, in the comprehensive evaluation, a sample that could be punched out in the punching test 1 was evaluated to have better breaking processability of the main body 110 than a sample that could be punched only in the punching test 2, and was evaluated as ⊙ in an evaluation result.

The evaluation method described above is similar for samples 100B, 100C, 100D, and 100E described later.

### <Test results of samples A1 to A15>

**[Table 1]**

| Test conditions of samples A1 to A15 | | | | Test results | | |
|---|---|---|---|---|---|---|
| Sample name | Heating conditions | | | Punching test 1 | Punching test 2 | Comprehensive evaluation |
| | Pressing pressure | Pressing time | Heating temperature | | | |
| | | | | (parallel) | (twist) | |
| | (MPa) | (min) | (°C) | | | |
| Sample A1 | Not heated by hot pressing | | | × | × | × |
| Sample A2 | 20 | 30 | 90 | × | × | × |
| Sample A3 | | | 110 | × | × | × |
| Sample A4 | | | 130 | × | × | × |
| Sample A5 | | | 140 | × | ○ | ○ |
| Sample A6 | | | 145 | × | ○ | ○ |
| Sample A7 | | | 150 | × | ○ | ○ |
| Sample A8 | | | 170 | × | ○ | ○ |
| Sample A9 | | | 180 | × | ○ | ○ |
| Sample A10 | | | 190 | ○ | ○ | ⊙ |
| Sample A11 | | | 200 | ○ | ○ | ⊙ |
| Sample A12 | | | 205 | ○ | ○ | ⊙ |
| Sample A13 | | | 210 | ○ | ○ | ⊙ |
| Sample A14 | 40 | | 200 | ○ | ○ | ⊙ |
| Sample A15 | 20 | | 220 | - | - | - |

From the test results presented in Table 1, for the sample A1 that had not been subjected to heat treatment by hot pressing, results of the punching test 1 and the punching test 2 were ×. Therefore, the sample A1 was evaluated as × in the comprehensive evaluation.

For each of the samples A2 to A4 under a condition of a heating temperature of 90°C or higher and 130°C or lower, results of the punching test 1 and the punching test 2 were ×. Therefore, the samples A2 to A4 were evaluated as × in the comprehensive evaluation as in the case of the sample A1.

For each of the samples A5 to A9 under a condition of a heating temperature of 140°C or higher and 180°C or lower, a result of the punching test 1 was ×, but a result of the punching test 2 was o. Therefore, the samples A5 to A9 were evaluated as ∘ in the comprehensive evaluation. From this result, it was confirmed that when heat treatment by hot pressing at 140°C or higher, which is the heating condition of the sample A5, was performed, a result of the punching test 2 was ∘ (o in comprehensive evaluation). In addition, from this, it was confirmed that the samples A5 to A9 had favorable breaking processability for application to an anastomotic portion.

For each of the samples A10 to A13 under a condition of a heating temperature of 190°C or higher and 210°C or lower, a result of the punching test 1 was ∘, and a result of the punching test 2 was also ∘. Therefore, the samples A10 to A13 were evaluated as ⊙ in the comprehensive evaluation. From this result, it was confirmed that when hot pressing at 190°C or higher, which is the heating condition of the sample A10, was performed, results of the punching test 1 and the punching test 2 were ∘ (⊙ in comprehensive evaluation). In addition, from this, it was confirmed that the samples A10 to A13 had very favorable breaking processability for application to an anastomotic portion.

In the sample A14, a heating temperature was set to 200°C as in the sample A11. In addition, in the sample A14, a pressing time was set to 30 minutes as in the sample A11. Note that, in the sample A14, a pressing pressure was set to 40 MPa, which is larger than 20 MPa in the sample A11. In the sample A14, a result of the punching test result 1 was ∘, and a result of the punching test 2 was ∘ (⊙ in comprehensive evaluation) as in the sample A11. From this, in the sample 100A, it can be estimated that a main factor of improving breaking processability is not the pressing pressure but the heating temperature.

In the sample A15, a pressing pressure and a pressing time were set as in the samples A2 to A13 (pressing pressure: 20 MPa, pressing time: 30 minutes). Note that, in the sample A15, a heating temperature was set to 220°C. In the sample A15, when hot pressing at a heating temperature of 220°C was performed, fibers of a nonwoven fabric constituting the main body 110 were melted and deformed into a film shape. Therefore, the through-holes 112 formed in the main body 110 disappeared. From this result, in the sample 100A, it was confirmed that when heating was performed at a heating temperature of 220°C or higher, an effect of promoting adhesion by the through-holes 112 might be impaired. From this, it was confirmed that the heating temperature was preferably lower than 220°C when the breakable portion 110A constituted by a heated portion was disposed in the sample 100A.

### <Samples B1 to B4>

Table 2 presents punching test results of medical members as samples B1 to B4.

The following medical members were prepared as samples B1 to B4. In the following description, the samples B1 to B4 are collectively referred to as "sample 100B".

### <Sample 100B>

- A sheet including the mesh-like main body 110 made of a polyglycolic acid (PGA) nonwoven fabric was prepared as the sample 100B.
- The sheet was constituted by one sheet made of a coarse nonwoven fabric. That is, the sample 100B is obtained by peeling one sheet from the above-described sample 100A constituted by two sheets. Therefore, in the sample 100B, an area occupied by the through-holes 112 in plan view of the main body 110 is larger than that of the sample 100A described above.
- Fig. 7 illustrates a schematic view in which a part of the sample 100B is enlarged. In the sample 100B, the through-hole 112 has a hole diameter of 0.2 mm to 1.4 mm and a wire diameter (pitch) of 0.1 mm to 0.2 mm. The sample 100B has a thickness of 0.04 mm to 0.11 mm. Note that the other configurations of the sample 100B are similar to those of the sample 100A described above.
- A heated portion that had been subjected to heat treatment in the main body 110 of the sample 100B or an arbitrary portion that had not been subjected to heat treatment in the main body 110 of the sample 100B was defined as the breakable portion 110A. Note that conditions and the like for forming the heated portion are similar to those of the sample 100A described above, and details thereof are presented in Table 2.

### <Test results of samples B1 to B4>

**[Table 2]**

| Test conditions of samples B1 to B4 | | | | Test results | | |
|---|---|---|---|---|---|---|
| Sample name | Heating conditions | | | Punching test 1 | Punching test 2 | Comprehensive evaluation |
| | Pressing pressure | Pressing time | Heating temperature | | | |
| | | | | (parallel) | (twist) | |
| | (MPa) | (min) | (°C) | | | |
| Sample B1 | Not heated by hot pressing | | | × | ○ | ○ |
| Sample B2 | 20 | 30 | 150 | ○ | ○ | ⊙ |
| Sample B3 | 20 | | 200 | ○ | ○ | ⊙ |
| Sample B4 | 40 | | 200 | ○ | ○ | ⊙ |

From the test results presented in Table 2, for the sample B1 that had not been subjected to heat treatment by hot pressing, a result of the punching test 1 was ×, but a result of the punching test 2 was ∘. Therefore, the sample B1 was evaluated as ∘ in the comprehensive evaluation. In the above-described sample A1 (not heated by hot pressing), since the results of the punching test 1 and the punching test 2 were ×, the sample A1 was evaluated as × in the comprehensive evaluation. From comparison between the test result of the sample B1 and the test result of the sample A1, it was confirmed that the sample 100B had higher breaking processability than the sample 100A, and had higher applicability to an anastomosis.

As presented in Table 2, in the samples B2 to B4 under a condition of a heating temperature of 150°C or higher and 200°C or lower, a result of the punching test 1 was ∘, and a result of the punching test 2 was also ∘. Therefore, the samples B2 to B4 were evaluated as ⊙ in the comprehensive evaluation. From this result, it was confirmed that when hot pressing at 150°C or higher was performed on the sample 100B, results of the punching test 1 and the punching test 2 were o (⊙ in comprehensive evaluation). In addition, from this, it was confirmed that the samples B2 to B4 had very favorable breaking processability for application to an anastomotic portion.

In comparison between the sample A1 that had not been subjected to heat treatment and the sample B1 that had not been subjected to heat treatment, the sample B1 exhibited higher breaking processability. In comparison between the sample A7 and the sample B2 that had been subjected to heat treatment under the same conditions, the sample B2 exhibited higher breaking processability. From this result, it was confirmed that, in the mesh-like main body 110 made of a polyglycolic acid (PGA) nonwoven fabric, breaking processability was higher as the size (corresponding to the hole diameter) of the through-hole 112 was larger and the thickness was smaller.

### <Samples C1 to C5>

Table 3 presents punching test results of the medical members as samples C1 to C5.

The following medical members were prepared as the samples C1 to C5. In the following description, the samples C1 to C5 are collectively referred to as "sample 100C".

### <Sample 100C>

- A sheet including the mesh-like main body 110 made of a polyglycolic acid (PGA) nonwoven fabric was prepared as the sample 100C.
- The sheet includes the plurality of through-holes 112 regularly arranged in the main body 110. The through-holes 112 are arranged in a staggered pattern so as to form an angle of 90° with an adjacent through-hole.

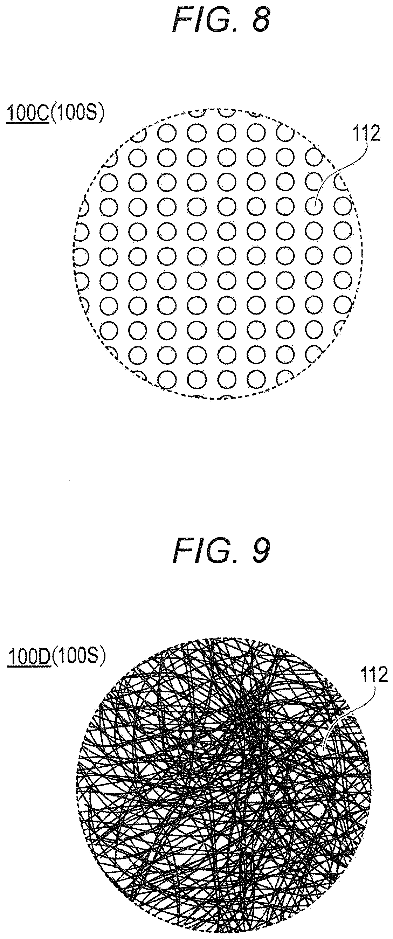

- Fig. 8 illustrates a schematic view in which a part of the sample 100C is enlarged. As presented in Table 3, in the sample 100C, the through-hole 112 had a hole diameter of 0.6 mm and a wire diameter (pitch) of 0.2 mm or 0.4 mm. The sample 100C has a thickness of 0.1 mm to 0.2 mm.
- The "number of stacked sheets" illustrated in Table 3 represents the number of the sheets stacked. "None in the number of stacked sheets" means one sheet, and the number of stacked sheets 3 and 6 mean that three sheets are stacked and six sheets are stacked, respectively. Note that the three or six sheets were arranged such that the through-holes 112 formed in the respective sheets overlapped each other in plan view.
- Similarly to each of the samples 100A and 100B described above, the breakable portion 110A was set to a heated portion that had been subjected to heat treatment in the main body 110 or an arbitrary portion that had not been subjected to heat treatment in the main body 110 of the sample 100C. Note that conditions and the like for forming the heated portion are similar to those of the samples 100A and 100B described above, and details thereof are presented in Table 3.

### <Test results of samples C1 to C5>

**[Table 3]**

| Test conditions of samples C1 to C5 | | | | | | | | Test results | |
|---|---|---|---|---|---|---|---|---|---|
| Sample name | Number of stacked sheets | Heating conditions | | | Mesh hole processing | | | Punching test 1 (parallel) | Comprehensive evaluation |
| | | Pressing pressure | Pressing time | Heating temperature | Hole diameter | Wire diameter | Arrangement of holes | | |
| | | (MPa) | (min) | (°C) | (mm) | (mm) | | | |
| Sample C1 | None | Not heated by hot pressing | | | 0.6 | 0.2 | 90° staggered pattern | ○ | ⊙ |
| Sample C2 | None | Not heated by hot pressing | | | | 0.4 | | ○ | ⊙ |
| Sample C3 | 3 | 40 | 20 | 150 | | 0.2 | | ○ | 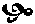 |
| Sample C4 | 3 | | | 170 | | 0.2 | | ○ | 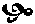 |
| Sample C5 | 6 | | | 170 | | 0.4 | | ○ | 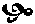 |

From the test results presented in Table 3, in the samples C1 to C5, a result of the punching test 1 was o. Therefore, the samples C1 to C5 were evaluated as ⊙ in the comprehensive evaluation. From this, it was confirmed that the sample 100C had very high breaking processability and high applicability to an anastomosis regardless of the number of stacked sheets, a condition for heat treatment, and other conditions (the size of the through-hole 112 and the like).

Note that, in the samples C1 to C5, since the result of the punching test 1 was o as described above, the punching test 2 was not performed.

### <Sample 100D>

Table 4 presents punching test results of the medical members as samples D1 to D3.

The following medical members were prepared as the samples D1 to D3. In the following description, the samples D1 to D3 are collectively referred to as "sample 100D".
- The sample 100D was constituted by a sheet including the mesh-like main body 110 made of a polyglycolic acid (PGA) nonwoven fabric similarly to the sample 100A.
- Fig. 9 illustrates a schematic view in which a part of the sample 100D is enlarged. The thickness of the main body 110, the size of the through-hole 112, the size of the pitch, and the like of the sample 100D are the same as those of the sample 100A.

A preparation method of the sample 100D is partially different from that of the sample 100A. Specifically, the samples D1 to D3 prepared as follows were prepared.
- Sample D1: The sample D1 was not subjected to heat treatment. Therefore, the sample D1 is substantially the same as the sample A1.
- Sample D2: The sample D2 was irradiation with radiation as pretreatment. Electron beam irradiation with a dose of 100 kGy was adopted.
- Sample D3: The sample D3 was irradiated with radiation and subjected to heat treatment by hot pressing in combination. Radiation irradiation conditions are similar to those of the sample D2. Heating conditions of the hot pressing were a pressing pressure of 20 MPa, a pressing time of 30 minutes, and a heating temperature of 150°C. Note that the heating conditions of the hot pressing are the same as those of the sample A7.

### <Test results of samples D1 to D3>

**[Table 4]**

| Test conditions of samples D1 to D3 | | Test results | | |
|---|---|---|---|---|
| Sample name | Heating conditions and the like | Punching test 1 | Punching test 2 | Comprehensive evaluation |
| | | (parallel) | (twist) | |
| Sample D1 | Not heated | × | × | × |
| Sample D2 | Radiation irradiation (pretreatment) | × | ○ | ○ |
| Sample D3 | Radiation irradiation (pretreatment) and hot pressing (pressing pressure: 20 MPa, pressing time: 30 minutes, heating temperature: 150°C) | ○ | ○ | ⊙ |

From the test results presented in Table 4, in the sample D1, a result of the punching test result 1 was ×, and a result of the punching test 2 was also × as in the sample A1. Meanwhile, in the sample D2 that had been subjected to pretreatment by radiation irradiation, a result of the punching test 1 was ×, but a result of the punching test 2 was ∘. Therefore, the sample D2 was evaluated as ∘ in the comprehensive evaluation. From comparison between the test result of the sample D1 and the test result of the sample D2, it was confirmed that breaking processability of the main body 110 could be improved by subjecting the sample 100D to pretreatment by radiation irradiation.

In the sample D3 that had been subjected to radiation irradiation and hot press heating, a result of the punching test 1 was ∘, and a result of the punching test 2 was also o. Therefore, the sample D3 was evaluated as ⊙ in the comprehensive evaluation. As a result of comparing the test result of the sample D3 with the test result of the sample D2, it was confirmed that breaking processability of the main body 110 was more effectively improved by subjecting the sample 100D to heat treatment of hot pressing together with radiation irradiation.

The sample A7 (see Table 1) that had been subjected to hot press heating under the same conditions as the hot press heating of the sample D3 which was evaluated as ⊙ in the comprehensive evaluation was evaluated as × in the comprehensive evaluation. In the sample D3, pretreatment by radiation irradiation is added as compared with the sample A7. From comparison between the test result of the sample D3 and the test result of the sample A7, it was confirmed that radiation irradiation had an effect of effectively improving breaking processability of the main body 110.

### <Sample 100E>

Table 5 presents punching test results of the medical member as sample 100E.

The following medical member was prepared as the sample 100E.

### <Sample 100E>

- As the sample 100E, a sheet including the mesh-like main body 110 made of glycolic acid/lactic acid polyester (A ratio of glycolic acid : a ratio of lactic acid polyester was 90 : 10.) was prepared. The sample 100E is a knit type sheet.

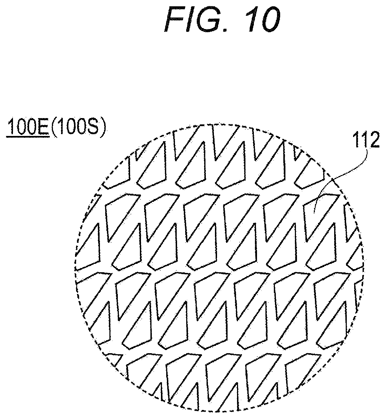

- Fig. 10 illustrates a schematic view in which a part of the sample 100E is enlarged. •The sheet used for the sample 100E includes the plurality of through-holes 112 regularly arranged. In the sample 100E, the through-hole 112 has a hole diameter of 0.3 mm to 0.7 mm and a wire diameter (pitch) of 0.1 mm to 0.2 mm. The sample 100E has a thickness of 0.2 mm.
- The sample 100E had not been subjected to heat treatment. Therefore, an arbitrary portion of the sample 100E was used as the breakable portion 110A.

### <Test results of sample 100E>

**[Table 5]**

| Test conditions of samples E | | Test results | |
|---|---|---|---|
| Sample name | Heating conditions | Punching test 1 (parallel) | Comprehensive evaluation |
| Sample E | Not heated | ○ | ⊙ |

As presented in Table 5, in the sample 100E, a result of the punching test 1 was ∘. In the sample 100E, since the result of the punching test 1 was ∘, it was expected that the result of the punching test result 2 would also be ∘. Therefore, the punching test 2 was not performed on the sample 100E, and the sample 100E was evaluated as ⊙ in the comprehensive evaluation. From this test result, it was confirmed that the sample 100E had very high breaking processability and high applicability to an anastomosis.

The sample 100E had not been subjected to heat treatment. From this, it can be confirmed that the sample 100E has a high breaking processability even when the main body 110 is not subjected to heat treatment.

From the results of the punching test 1 and/or the punching test 2 performed on the samples 100A to 100E, it was confirmed that breaking processability of the main body 110 of the medical member 100 was affected by a material quality of a material constituting the main body 110, presence or absence of heat treatment, a heating method, a heating temperature, arrangement of the through-holes 112, and the like.

On the basis of the results of the punching test 1 and/or the punching test 2, the inventors measured "strength (breaking strength)" and "compression elongation percentage (elastic modulus indicating flexibility in plane direction)" of each of the samples 100A to 100E, considered to have an influence on breaking processability.

Hereinafter, test conditions of the breaking strength test and the elastic modulus measurement test performed on the samples 100A to 100E, and test results thereof will be described.

### <Breaking strength measurement test>

For the breaking strength measurement test, a known creep meter was used.

Each of the samples was cut into a square shape having a side of 15 mm and set in a predetermined fixing jig. The fixing jig has an opening that exposes a center portion of the sample in the plane direction. The fixing jig to which the sample was set was attached to a lower part of the creep meter.

A cylindrical jig having φ of 3 mm (≈ 7 mm²) was used as a pushing jig that transmits a pushing force to the sample through the opening of the fixing jig. The cylindrical jig was attached to an upper part of the creep meter.

The cylindrical jig was caused to approach the opening of the fixing jig, and a pushing force of a constant speed (5 mm/sec) was applied to the sample through the opening of the fixing jig. A maximum load obtained in this test was calculated as a breaking strength of the sample (breaking strength in a direction orthogonal to the plane direction). For the calculation of the breaking strength, commercially available software for breaking strength analysis was used.

### <Elastic modulus measurement test>

In the elastic modulus measurement test, a known creep meter was used as in the breaking strength measurement test. Description of similar conditions to those in the breaking strength measurement test will be omitted.

In the elastic modulus test, a pushing force of a constant load (0.1 N) was applied to the sample through the opening of the fixing jig. A maximum value of measured values of a strain ratio obtained in this test was calculated as a compression elongation percentage. For the calculation of the compression elongation percentage, commercially available software for elastic analysis was used.

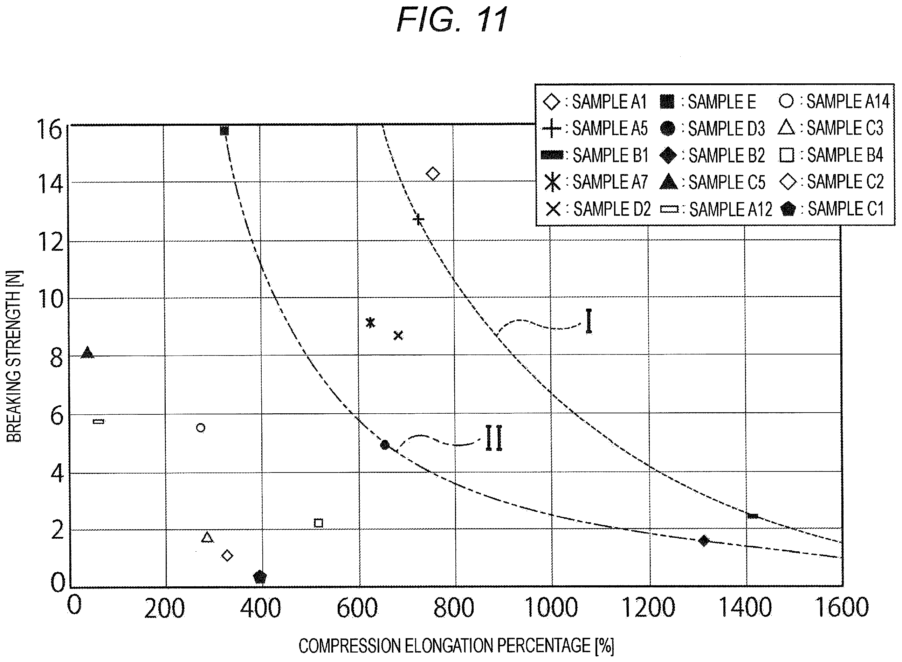

Fig. 11 illustrates a relationship between a breaking strength (Y-axis) and a compression elongation percentage (X-axis) of each of the samples obtained by the breaking strength measurement test and the elastic modulus measurement test.

The samples that were subjected to the breaking strength measurement test and the elastic modulus measurement test are as follows.
Sample A1 (punching test 1: ×, punching test 2: ×, comprehensive evaluation: ×)
Sample A5 (punching test 1: ×, punching test 2: o, comprehensive evaluation: ∘)
Sample B1 (punching test 1: ×, punching test 2: ∘, comprehensive evaluation: ∘)
Sample A7 (punching test 1: ×, punching test 2: ∘, comprehensive evaluation: ∘)
Sample D2 (punching test 1: ×, punching test 2: ∘, comprehensive evaluation: ∘)
Sample E (punching test 1: ∘, punching test 2: o, comprehensive evaluation: ⊙)
Sample D3 (punching test 1: ∘, punching test 2: o, comprehensive evaluation: ⊙)
Sample B2 (punching test 1: ∘, punching test 2: o, comprehensive evaluation: ⊙)
Sample C5 (punching test 1: ∘, punching test 2: o, comprehensive evaluation: ⊙)
Sample A12 (punching test 1: ∘, punching test 2: o, comprehensive evaluation: ⊙)
Sample A14 (punching test 1: ∘, punching test 2: o, comprehensive evaluation: ⊙)
Sample C3 (punching test 1: ∘, punching test 2: o, comprehensive evaluation: ⊙)
Sample B4 (punching test 1: ∘, punching test 2: o, comprehensive evaluation: ⊙)
Sample C2 (punching test 1: ∘, punching test 2: o, comprehensive evaluation: ⊙)
Sample C1 (punching test 1: ∘, punching test 2: o, comprehensive evaluation: ⊙)

The inventors have found that the quality of the breaking processability of the medical member 100 depends on the breaking strength and the compression elongation percentage among the physical properties of the main body 110. This is based on a finding that, as described at the beginning of the present specification, in a case where the main body 110 has low flexibility, it is difficult to extend the main body 110, and therefore, when a cutter or the like is pressed against the main body 110, a force pressing the cutter can be sufficiently transmitted in the thickness direction of the main body 110 (direction intersecting with the plane direction of the main body 110), and a finding that in a case where the main body 110 has low strength, when the cutter is pressed against the main body 110, a cutting edge of the cutter can be easily bitten into the main body 110. From this, it is concluded that the main body 110 has high breaking processability by including the breakable portion 110A formed so as to have a lower "compression elongation percentage" and a lower "breaking strength".

In addition, from the finding that "The sample A1 (× in comprehensive evaluation) is not suitable for an anastomosis, and the sample A5 (o in comprehensive evaluation) and the sample B1 (o in comprehensive evaluation) are suitable for an anastomosis." obtained from the results of the punching test 1 and the punching test 2 described above, the inventors have found that the quality of the breaking processability of the main body 110 can be defined on the basis of the "chart indicating a relationship between a breaking strength (Y axis) and a compression elongation percentage (X axis)" illustrated in Fig. 11.

Specifically, on the chart illustrated in Fig. 11, the inventors have found that the breakable portion 110A having physical properties in a predetermined range expressed by a power approximate expression representing a curve I (broken line I) passing through the sample A5 and the sample B1 has high applicability to an anastomosis, that is, is evaluated as ∘ in the comprehensive evaluation of the breaking processability.

In addition, on the chart illustrated in Fig. 11, the inventors have found that the breakable portion 110A having physical properties in a predetermined range expressed by a power approximate expression representing a curve II (twodot chain line II) passing through the sample E, the sample D3, and the sample B2 has very high applicability to an anastomosis, that is, is evaluated as ⊙ in the comprehensive evaluation of the breaking processability.

The curve I represented on the chart illustrated in Fig. 11 can be expressed by a power approximate expression taking values of the sample A5 and the sample B1. In addition, the curve II can be expressed by a power approximate expression taking values of the sample E, the sample D3, and the sample B2.

Specifically, when the breaking strength of the main body 110 is represented by Y [N], and
the compression elongation percentage of the main body 110 is represented by X [%],
the curve I and the curve II are expressed by a power approximate expression satisfying the following formulas (1) to (4): $Y < 244734 {\left(X-Z\right)}^{- 1.665}$ $0 \leq Z < 408.216$ $X - Z > 0$ $39 \leq X$

Here, when Z = 351.466 is satisfied, the curve I is represented on the chart of Fig. 11. When Z = 0 is satisfied, the curve II is represented on the chart of Fig. 11.

When X - Z > 0 in the formula (3) is not satisfied, a value of Y represented by formula (1) cannot be calculated. In addition, 39 ≤ X in the above formula (4) is a variable determined by a lower limit value (measured value of the sample C5) measured in the performed breaking strength measurement test and elastic modulus measurement test.

In the chart illustrated in Fig. 11, in a range (region) where a value of Y is smaller than each value in the curve II, the comprehensive evaluation of the punching tests of the main body 110 is ⊙. Therefore, all of the samples C5, A12, A14, C3, B4, C2, and C1 for which the comprehensive evaluation of the punching tests is ⊙ are included in the above range.

In addition, in a range where a value of Y is larger than each value on the curve II and equal to or less than each value on the curve I (a region sandwiched between the curve I and the curve II on the chart of Fig. 11), the comprehensive evaluation of the punching tests of the main body 110 is o. All of the samples A5, B1, A7, and D2 for which the comprehensive evaluation of the punching test is ∘ are included in the above range.

From the chart illustrated in Fig. 11, when a value of Z is 408.216 or less, the main body 110 is expected to have breaking processability to such an extent that the comprehensive evaluation of the punching tests is o.

Note that the condition of 0 ≤ Z < 408.216 indicated in formula (2) is a condition for excluding the sample A1 evaluated as × in the comprehensive evaluation of the punching tests.

In addition, in formula (2), the comprehensive evaluation of the punching tests is o in a range where 0 ≤ Z ≤ 351.466 is satisfied (a range where a value of Y is equal to or less than the curve I), and the comprehensive evaluation of the punching tests is ⊙ in a range where Z = 0 is satisfied (a range where a value of Y is equal to or less than the curve II). Therefore, it can be confirmed that the breaking processability of the main body 110 defined by formulas (1) to (4) depends on a value of Z.

A determination coefficient of the power approximate expression of formula (1) representing the curve I in Fig. 11 is 0.9144. A determination coefficient of the power approximate expression of formula (1) representing the curve II in Fig. 11 is 0.9997. From these values of the determination coefficient, it is inferred that the power approximate expression (1) representing the curve I and the curve II has statistically high reliability.

**[Table 6]**

| Sample name | Comprehensive evaluation of punching tests | Breaking strength (N) | | compression elongation percentage (%) | |
|---|---|---|---|---|---|
| | | Average value | Standard deviation | Average value | Standard deviation |
| Sample A1 | × | 14.3 | 3.40 | 757 | 19 |
| Sample A5 | ○ | 12.7 | 0.11 | 726 | 33 |
| Sample D2 | | 8.70 | 1.60 | 683 | 66 |
| Sample B1 | | 2.40 | 0.38 | 1414 | 56 |
| Sample E | ⊙ | 15.8 | 0.20 | 329 | 43 |
| Sample D3 | | 4.90 | 0.60 | 655 | 66 |
| Sample B2 | | 1.60 | 0.36 | 1312 | 119 |

Table 6 presents data in which test results of the breaking strength measurement test and the elastic modulus measurement test of the sample A1 (× in comprehensive evaluation of punching tests) and the samples A5, D2, B1, E, D3, and B2 used for calculation of the power approximate expression representing the curves I and II are summarized. The "average value" presented in Table 6 is an average value of test results obtained by performing a test three times for each of the samples. The "standard deviation" is a standard deviation of test results obtained by performing a test three times for each of the samples.

From the results presented in Table 6, it is considered that when the main body 110 has a breaking strength of 16.0 N or less which is substantially the same as the test result of the sample E, breaking processability is improved. Meanwhile, when the breaking strength of the main body 110 is less than 0.2 N, handleability by a surgeon is significantly impaired. Therefore, it can be said that the breaking strength of the main body 110 is preferably 0.2 N or more and 16 N or less.

From the results presented in Table 6, it can be said that the main body 110 preferably has a compression elongation percentage of 1414% or less which is the same as the test result of the sample B1. Note that the compression elongation percentage of the main body 110 is 39% or more which is a measured value (see the chart illustrated in Fig. 11) of the sample C5 (⊙ in comprehensive evaluation of punching tests), and is more preferably 1312% or less which is a measured value of the sample B2 (⊙ in comprehensive evaluation of punching tests) from a viewpoint of imparting better breaking processability.

### <Operation and Effect>

As described above, the medical member 100 according to the present embodiment includes the mesh-like main body 110 that has the plurality of through-holes 112, induces expression of a biological component by being applied to an anastomotic portion of a biological organ, and promotes adhesion of the anastomotic portion by the induced biological component passing through the through-holes 112 and being accumulated, in which
the main body 110 includes,
when a breaking strength of the main body 110 in a direction intersecting with a plane direction is represented by Y [N], and
a compression elongation percentage of the main body 110 in the plane direction is represented by X [%],
a breakable portion having a relationship between the breaking strength and the compression elongation percentage, represented by the following formulas (1) to (2): $Y \leq 244734 {X}^{- 1.665}$ $39 \leq X \leq 1312$

According to the present embodiment, it is possible to provide the medical member 100 having appropriate flexibility and strength for application to an anastomotic portion and having effectively improved breaking processability.

In addition, when 0 ≤ Z ≤ 351.466 is satisfied in the above formula (2), the breaking processability of the medical member 100 is effectively improved.

In addition, when 0 = Z is satisfied in the above formula (2), the breaking processability of the medical member 100 is more effectively improved.

In addition, when the breaking strength of the medical member 100 is 16 N or less, the breaking processability is effectively improved.

In addition, when the medical member 100 has a compression elongation percentage of 1414% or less, breaking processability is effectively improved.

In addition, at least a part of the breakable portion 110A formed in the main body 110 of the medical member 100 is disposed at a position closer to the center portion O1 of the main body 110 in the plane direction than an outer peripheral portion of the main body 110. According to the medical member 100 configured in this manner, when an anastomosis is performed using the joining device 700 (see Fig. 14), the main body 110 can be smoothly punched out in a predetermined range including the center portion O1 of the main body 110 in the plane direction. Therefore, the medical member 100 can be suitably applied to an anastomosis between biological organs in which a lumen is formed, such as a large intestine anastomosis.

In addition, when polyglycolic acid is contained in a constituent material of the breakable portion 110A, the main body 110 of the medical member 100 has effectively improved breaking processability.

In addition, when polyglycolic acid is contained in a constituent material of the breakable portion 110A and the breakable portion 110A is made of a nonwoven fabric, the breaking processability of the main body 110 of the medical member 100 is further improved.

In addition, when the breakable portion 110A is constituted by a heated portion that has been subjected to heat treatment, the breaking processability of the main body 110 of the medical member 100 is further effectively improved.

### <Embodiment of treatment method (large intestine anastomosis)>

Next, a treatment method using a medical member (adhesion promoting device) will be described.

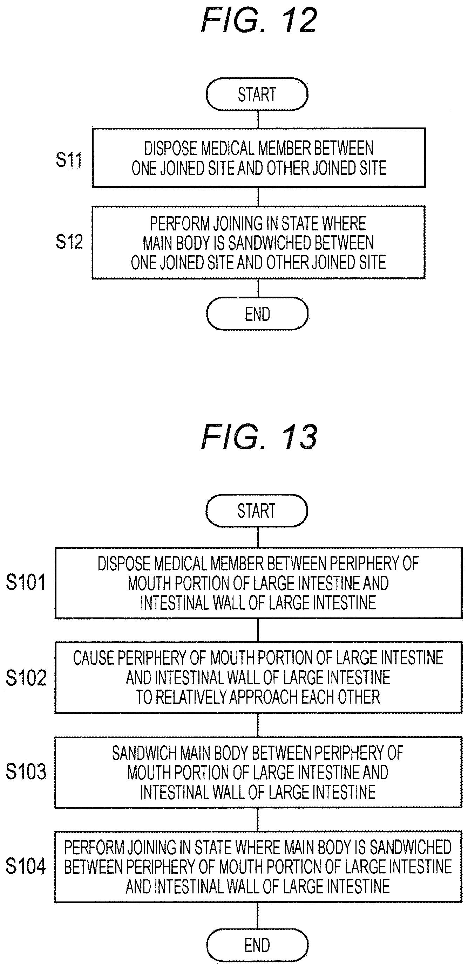

Fig. 12 is a flowchart illustrating steps of the treatment method using a medical member.

The treatment method includes: disposing a medical member including a main body that promotes adhesion of a biological tissue between one joined site of a biological organ and the other joined site to be joined (S11); and joining the one joined site and the other joined site in a state where at least a part of the main body of the medical member is disposed between the one joined site and the other joined site (S12).

The biological organ and the joined site in the biological organ, to be joined by the treatment method of the present embodiment are not particularly limited, and can be arbitrarily selected. Note that, in the following description, a large intestine anastomosis will be described as an example.

As a medical member used in a technique described below, for example, a medical member having the structure illustrated in Fig. 1 can be selected. Note that a specific configuration of the medical member is not particularly limited.

In addition, in the following description, a use example of the medical member will be described through a representative example that can be suitably used for a large intestine anastomosis. In the technique described below, detailed description of known technique steps, a known medical device, a known medical tool, and the like will be appropriately omitted.

Hereinafter, in the description of the present specification, "disposing a medical member between biological organs" means at least one of disposing the medical member in a state where the medical member is in direct or indirect contact with the living organs, disposing the medical member in a state where a spatial gap is formed between the medical member and the living organs, and disposing the medical member in both the states (for example, the medical member is disposed in a state where the medical member is in contact with one biological organ and not in contact with the other biological organ). In addition, in the description of the present specification, a "periphery" does not define a strict range (region), and means a predetermined range (region) as long as a purpose of treatment (joining of biological organs) can be achieved. In addition, the order of the technique steps described in each treatment method can be appropriately changed as long as a purpose of treatment can be achieved. In addition, in the description of the present specification, "causing objects to relatively approach each other" means both causing two or more objects as approaching targets to approach each other and causing only one object approach the other object.

### <Embodiment of treatment method (large intestine anastomosis)>

Fig. 13 is a flowchart illustrating steps of an embodiment of a treatment method (large intestine anastomosis). Figs. 14 to 16 are diagrams for describing a large intestine anastomosis.

In the treatment method according to the present embodiment, a biological organ to be joined is a large intestine cut at the time of excision of a cancer tumor. Specifically, the biological organ to be joined includes a mouth side A1 of the cut large intestine and an anal side A2 of the cut large intestine. In the following description, steps of joining a periphery of a mouth portion of the mouth side A1 of the cut large intestine (one joined site) and a part of an intestinal wall of the anal side A2 of the cut large intestine (the other joined site) will be described.

As illustrated in Fig. 13, the treatment method according to the present embodiment includes: disposing a medical member between a periphery of a mouth portion of a large intestine and an intestinal wall of the large intestine (S101); causing the periphery of the mouth portion of the large intestine and the intestinal wall of the large intestine to relatively approach each other (S102); sandwiching a main body of the medical member between the periphery of the mouth portion of the large intestine and the intestinal wall of the large intestine (S103); and performing joining in a state where the main body of the medical member is sandwiched between the periphery of the mouth portion of the large intestine and the intestinal wall of the large intestine (S104).

Next, the treatment method according to the present embodiment will be specifically described with reference to Figs. 14 to 16.

First, a surgeon forms a port (an introduction portion for moving various medical devices and the like into and out of a living body) in a peripheral portion of a navel of a patient, and inflates an abdomen of the patient.

Next, the surgeon forms an incision (not illustrated) around the navel, takes out an affected part of the mouth side A1 from the incision, and inserts the first engagement tool 710 of the joining device 700 into the mouth side A1 of the large intestine. The surgeon inserts the engaged portion 711 of the first engagement tool 710 into the mouth side A1 of the large intestine, and performs purse-string suture in a state where the engaged portion 711 protrudes to form a suture portion A11. An outer surface of the suture portion A11 has an uneven shape due to the suture.

As the joining device 700, for example, a known device used for a large intestine anastomosis can be used. With engagement of the first engagement tool 710 and the second engagement tool 720, the joining device 700 can excise a biological tissue disposed between the first engagement tool 710 and the second engagement tool 720, and suture a periphery of the excised biological tissue circumferentially with staples. The first engagement tool 710 is, for example, a tool including the cylindrical engaged portion 711, and the second engagement tool 720 is, for example, a tool including an engagement pin 721 to be inserted into and engaged with the engaged portion 711 of the first engagement tool 710.

Next, as illustrated in Fig. 14, the surgeon disposes the medical member 100 in the biological tissue of the mouth side A1 of the large intestine (S101). When disposing the medical member 100, the surgeon causes the engaged portion 711 included in the first engagement tool 710 to pass through the hole portion 114 (see Fig. 1) formed in the main body 110 of the medical member 100. At this time, the surgeon can set the medical member 100 such that the back surface 113 of the medical member 100 comes into contact with an outer surface of the suture portion A11.

Next, the surgeon introduces the mouth side A1 of the large intestine in which the medical member 100 is disposed into a body of the patient from the incision.

Next, the surgeon disposes the second engagement tool 720 of the joining device 700 in the anal side A2 of the large intestine. As the second engagement tool 720 is disposed in (inserted into) the anal side A2 of the large intestine, a through-hole A21 is formed in the anal side A2 of the large intestine. Note that a specific timing of forming the through-hole A21 is not particularly limited.

The surgeon can dispose the medical member 100 between the mouth side A1 of the large intestine and the anal side A2 of the large intestine by engaging the engaged portion 711 of the first engagement tool 710 with the engagement pin 721 of the second engagement tool 720 while maintaining a state of holding the main body 110 with respect to the mouth side A1 of the large intestine. Specifically, as illustrated in Fig. 15, the surgeon causes the first engagement tool 710 and the second engagement tool 720 to relatively approach each other and engages the first engagement tool 710 and the second engagement tool 720 with each other while maintaining a state of holding the medical member 100 with respect to the suture portion A11 of the mouth side A1 of the large intestine (S102).

Next, the surgeon sandwiches a periphery of the mouth portion of the mouth side A1 of the large intestine, the main body 110 of the medical member 100, and a peripheral portion of the through-hole A21 formed in the intestinal wall of the anal side A2 of the large intestine between the first engagement tool 710 and the second engagement tool 720 (S103). Then, the surgeon cuts a part of the mouth side A1 of the large intestine, the main body 110 of the medical member 100, and a part of the anal side A2 of the large intestine, sandwiched between the first engagement tool 710 and the second engagement tool 720 so as to be punched out by the joining device 700. In addition, at this time, the surgeon joins a periphery of the excised site with staples (not illustrated) by operating the joining device 700 (S104).

As described above, in the medical member 100 according to the present embodiment, the breakable portion 110A having improved breaking processability is formed in the main body 110. Therefore, the surgeon can smoothly punch out a part of the main body 110. As a result, it is possible to prevent occurrence of punching failure in which fraying occurs in the main body 110 when the main body 110 is punched out.

Next, as illustrated in Fig. 16, the surgeon takes out the joining device 700 from the anal side A2 of the large intestine to the outside of the living body through an anus, for example. At this time, the surgeon can take out a part of the mouth side A1 of the large intestine located on an inner side of a second region E2 punched out by the joining device 700, a part of the main body 110 of the medical member 100 (a part including the breakable portion 110A at least as a part thereof), and a part of the anal side A2 of the large intestine to the outside of the living body together with the joining device 700. Meanwhile, the pressure resistance improving portion 110B disposed on an outer peripheral portion side of the second region E2 is indwelled in the living body in a state of being sandwiched between a periphery of the mouth portion of the mouth side A1 of the large intestine and the intestinal wall of the anal side A2 of the large intestine. Therefore, the pressure resistance improving portion 110B of the main body 110 can effectively exhibit a function of promoting adhesion between the periphery of the mouth portion of the mouth side A1 of the large intestine to be joined and the intestinal wall of the anal side A2 of the large intestine to be joined.

According to such a treatment method, a risk of suture failure after an anastomosis (for example, a large intestine anastomosis) can be reduced by a simple method for sandwiching the main body of the medical member between one joined site and the other joined site.

Although the medical member according to the present invention has been described above through the embodiment, the present invention is not limited only to the contents described in the embodiment, and can be appropriately changed on the basis of the description in the claims.

For example, the biological organ to be joined, the joined site, the specific technique steps, and the like are not limited to those described in the embodiment. In addition, the material quality, size, shape, specific structure, and the like of the medical tool are not particularly limited as long as the main body included in the medical member has a function of promoting adhesion of a biological tissue at a joined site.

### Reference Signs List

- 100: medical member
- 110: main body
- 110A: breakable portion
- 110B: pressure resistance improving portion
- 112: through-hole
- 114: hole portion
- 700: joining device
- 710: first engagement tool
- 711: engaged portion
- 720: second engagement tool
- 721: engagement pin
- A1: mouth side
- A11: suture portion
- A2: anal side
- A21: through-hole
- D: hole diameter
- O1: center portion of main body
- P: pitch of through-hole
- T: thickness of main body

## Claims

1. A medical member (100) comprising a mesh-like main body (110) that has a plurality of through-holes (112, A21), induces expression of a biological component by being applied to an anastomotic portion of a biological organ, and promotes adhesion of the anastomotic portion by the induced biological component passing through the through-holes (112, A21) and being accumulated,
**characterized in that**
the main body (110) includes,
when a breaking strength of the main body (110) in a direction perpendicular to a plane defined by the planar shaped main body (110) is represented by Y [N], and
a compression elongation percentage of the main body (110) in the plane direction is represented by X [%],
a breakable portion (110A) having a relationship between the breaking strength and the compression elongation percentage, represented by the following formulas (1) to (2): $Y \leq 244734 {X}^{- 1.665}$ $39 \leq X \leq 1312$

2. The medical member (100) according to claim 1, wherein the breaking strength is 16 [N] or less.

3. The medical member (100) according to any one of claims 1 to 2, wherein at least a part of the breakable portion (110A) is disposed at a position closer to a center portion of the main body (110) in the plane direction than an outer peripheral portion of the main body (110).

4. The medical member (100) according to any one of claims 1 to 3, wherein a constituent material of the breakable portion (110A) contains polyglycolic acid.

5. The medical member (100) according to claim 4, wherein the breakable portion (110A) is made of a nonwoven fabric.

6. The medical member (100) according to claim 5, wherein the breakable portion (110A) is constituted by a heated portion that has been subjected to heat treatment.

## Patentansprüche

1. Medizinisches Element (100), umfassend einen maschenartigen Hauptkörper (110), der eine Vielzahl von Durchgangslöchern (112, A21) aufweist, die Expression einer biologischen Komponente induziert, indem es an einem Anastomosenabschnitt eines biologischen Organs angebracht wird, und die Adhäsion des Anastomosenabschnitts fördert, indem die induzierte biologische Komponente durch die Durchgangslöcher (112, A21) hindurchtritt und sich dort ansammelt,
**dadurch gekennzeichnet, dass**
der Hauptkörper (110),
wenn eine Bruchfestigkeit des Hauptkörpers (110) in einer Richtung senkrecht zu einer durch den flächig geformten Hauptkörper (110) definierten Ebene durch Y [N] dargestellt wird, und
ein prozentualer Dehnungswert bei Druckbeanspruchung des Hauptkörpers (110) in der Ebenenrichtung durch X [%] dargestellt wird,
einen zerbrechbaren Abschnitt (110A) umfasst, der eine Beziehung zwischen der Bruchfestigkeit und dem prozentualen Dehnungswert bei Druckbeanspruchung aufweist, die durch die folgenden Formeln (1) bis (2) dargestellt wird: $Y \leq 244734 {X}^{- 1.665}$ $39 \leq X \leq 1312$

2. Medizinisches Element (100) nach Anspruch 1, wobei die Bruchfestigkeit 16 [N] oder weniger beträgt.

3. Medizinisches Element (100) nach einem der Ansprüche 1 bis 2, wobei mindestens ein Teil des zerbrechbaren Abschnitts (110A) in der Ebenenrichtung in einer Position näher an einem Mittelabschnitt des Hauptkörpers (110) angeordnet ist als ein äußerer Umfangsabschnitt des Hauptkörpers (110).

4. Medizinisches Element (100) nach einem der Ansprüche 1 bis 3, wobei ein konstituierendes Material des zerbrechbaren Abschnitts (110A) Polyglykolsäure enthält.

5. Medizinisches Element (100) nach Anspruch 4, wobei der zerbrechbare Abschnitt (110A) aus einem Vliesmaterial hergestellt ist.

6. Medizinisches Element (100) nach Anspruch 5, wobei der zerbrechbare Abschnitt (110A) durch einen erwärmten Abschnitt gebildet wird, der einer Wärmebehandlung unterzogen wurde.

## Revendications

1. Élément médical (100) comprenant un corps principal maillé (110) qui présente une pluralité de trous traversants (112, A21), induit l'expression d'un composant biologique par application sur une partie anastomotique d'un organe biologique, et favorise l'adhérence de la partie anastomotique par le passage du composant biologique induit à travers les trous traversants (112, A21) et s'accumulant,
**caractérisé en ce que**
le corps principal (110) inclut,
lorsqu'une résistance à la rupture du corps principal (110) dans une direction perpendiculaire à un plan défini par le corps principal de forme plane (110) est représentée par Y [N], et
un pourcentage d'allongement-compression du corps principal (110) dans la direction du plan est représenté par X [%],
une partie cassable (110A) présentant une relation entre la résistance à la rupture et le pourcentage d'allongementcompression, représentée par les formules (1) à (2) suivantes : $Y \leq 244 {743 X}^{- 1 , 665}$ $39 \leq X \leq 1 312$

2. Élément médical (100) selon la revendication 1, dans lequel la résistance à la rupture est inférieure ou égale à 16 [N].

3. Élément médical (100) selon l'une quelconque des revendications 1 à 2, dans lequel au moins une partie de la partie cassable (110A) est disposée en une position plus proche d'une partie centrale du corps principal (110) dans la direction du plan que d'une partie périphérique extérieure du corps principal (110).

4. Élément médical (100) selon l'une quelconque des revendications 1 à 3, dans lequel un matériau constitutif de la partie cassable (110A) contient de l'acide polyglycolique.

5. Élément médical (100) selon la revendication 4, dans lequel la partie cassable (110A) est constituée d'un tissu non tissé.

6. Élément médical (100) selon la revendication 5, dans lequel la partie cassable (110A) est composée d'une partie chauffée ayant subi un traitement thermique.
